# FASCICULE DE BREVET EUROPEEN

(11) **EP 2 461 816 B1**
(45) Date de publication et mention de la délivrance du brevet: **11.11.2015**
(21) Numéro de dépôt: 10732700.9
(22) Date de dépôt: 13.07.2010
(51) Int. Cl.: A61K 33/00, A61P 25/00

(54) **UTILISATION DU XÉNON POUR TRAITER L'HYPERSENSIBILITÉ À LA DOULEUR**
VERWENDUNG VON XENON ZUR BEHANDLUNG VON SCHMERZÜBEREMPFINDLICHKEIT
USE OF XENON FOR TREATING HYPERSENSITIVITY TO PAIN

(30) Priorité: 04.08.2009 FR 0955476
(43) Date de publication de la demande: 13.06.2012
(73) Titulaire: L'Air Liquide Société Anonyme pour l'Etude et l'Exploitation des Procédés Georges Claude, 75007 Paris (FR); Université Bordeaux Segalen, 33076 Bordeaux Cedex (FR)
(72) Inventeur: BESSIERE, Baptiste, F-92130 Issy les Moulineaux (FR); SIMONNET, Guy, F-33200 Bordeaux (FR)
(74) Mandataire: Pittis, Olivier
(86) Numéro de dépôt international: PCT/EP2010/060021
(87) Numéro de publication internationale: WO 2011/015428

(56) Documents cités:
- WO-A2-02/22141
- FR-A1- 2 914 633
- MA DAQING ET AL: "Xenon exerts age-independent antinociception in Fischer rats" mai 2004 (2004-05), ANESTHESIOLOGY (HAGERSTOWN), VOL. 100, NR. 5, PAGE(S) 1313-1318 , XP008120757 ISSN: 0003-3022 page 1313, colonne 1, alinéa 2 page 1313, colonne 2, alinéa 2
- PETERSEN-FELIX S ET AL: "Comparison of the analgesic potency of xenon and nitrous oxide in humans evaluated by experimental pain" BRITISH JOURNAL OF ANAESTHESIA, vol. 81, no. 5, novembre 1998 (1998-11), pages 742-747, XP002576336 ISSN: 0007-0912

## Description

La présente invention porte sur l'utilisation de xénon pour le traitement ou la prévention de l'hypersensibilité à la douleur et par conséquent la chronicisation de la douleur.

Il est connu qu'à la suite d'une inflammation, d'une lésion tissulaire ou nerveuse, un processus de sensibilisation à la douleur va se développer et conduire à une hypersensibilité à la douleur. Ceci se traduit par l'apparition d'hyperalgésie, c'est-à-dire une réponse exagérée à une stimulation nociceptive, et/ou d'allodynie, c'est-à-dire une sensation douloureuse évoquée par un stimulus non nociceptif, et à une douleur persistante, comme rappelé par les articles de C. Woolf et al, Neuronal plasticity: increasing the gain in pain, Science, 288, 1765-9 (2000) ; J. Scholz et al, Can we conquer pain?, Nat Neurosci. 5 Suppl, 1062-7 (2002) ; et O. Wilder-Smith et al, Postoperative hyperalgesia: its clinical importance and relevance, Anesthes., 104, 601-7 (2006).

Au niveau du système nerveux central (SNC), plusieurs études expérimentales et cliniques ont montré un rôle critique des amino-acides excitateurs dans la sensibilisation à la douleur post-traumatique via les récepteurs N-methyl-D-aspartate (NMDA) comme rappelé par la revue B. Chizh et al., NMDA antagonists and neuropathic pain -Multiple drug targets and multiple uses, Current Pharmaceutical Design, 11, 2977-94 (2005).

Le phénomène de sensibilisation à la douleur subséquemment à une inflammation, une lésion tissulaire ou nerveuse, s'exprimant par une hyperalgésie ou une allodynie et une douleur accrue, et est désormais considérée comme le mécanisme clé à l'origine du développement de douleurs postopératoires et chroniques, comme rappelé par F. Perkins et al, Chronic pain as an outcome of surgery. A review of predictive factors. Anesthesiology 93, 1123-33 (2000); ou par W. Macrae, Chronic pain after surgery. British Journal of Anaesthesiology, 87, 88-98 (2001).

En particulier, durant les anesthésies générales, il est habituel d'administrer des hypnotiques volatiles ou intraveineux aux patients, ou des substances analgésiques intraveineuses ou opioïdes. On peut citer notamment les produits anesthésiques suivants qui sont couramment utilisés en anesthésie, tels que fentanyl, alfentanyl, sufentanyl, remifentamil ou autres opioïdes...

Or, il a été constaté que ces opioïdes volatiles ou intraveineux induisent et/ou amplifient les hyperalgésies post-opératoires, qui se traduisent par une sensibilité à la douleur des patients d'intensité accrue après l'opération. Il a d'ailleurs été montré que cette plus grande sensibilité à la douleur induite par les opioïdes est lié à l'hyperactivité du récepteur NMDA comme rappelé par les articles de Simonnet et al., Opioid-induced Hyperalgesia : abnormal or normal pain ? Neuroreport, 14, 1-7, (2003) ; et Angst et al., Opioid-induced Hyperalgesia, Anesthesiology, 104:570-87. (2006).

Cet effet est notable durant une période longue, pouvant aller notamment jusqu'à 1 an, et conduit donc à une diminution de la qualité de vie des patients puisque ceux-ci seront plus sensibles à la douleur durant tout ce temps.

Pour tenter d'y remédier, le document FR-A-2914633 a proposé l'utilisation d'un mélange gazeux contenant de l'oxygène (O₂) et de 2 à 10 % en volume de xénon (Xe) pour prévenir ou minimiser les hyperalgésies post-opératoires chez l'homme.

En effet, le xenon qui est un antagoniste des récepteurs NMDA, comme enseigné par N. Franks et al, How does xenon produce anaesthesia ? Nature, 396, 324. (1998), a un effet analgésique aigu, comme rappelé par D. Ma et al. Xenon exerts age-independent antinociception in Fischer rats, Anesthesiology. 100, 1313-8. (2004), et S. Petersen-Felix et al, Comparison of the analgesic potency of xenon and nitrous oxide in humans evaluated by experimental pain; Br J Anaesth. 81, 742-7 (1998).

De là, étant donné que les récepteurs NMDA ont un rôle critique majeur dans la sensibilisation à la douleur induite par les traumas et amplifiée par les opioïdes, on peut utiliser cette propriété pour prévenir ou traiter l'hypersensibilité à la douleur, grâce aux propriétés antagonistes des récepteurs NMDA du xénon utilisé en tant qu'agent pharmacologique.

Toutefois, la solution proposée par FR-A-2914633 n'est pas idéale car les concentrations en xénon sont trop faibles et ne permet donc pas d'obtenir un traitement réellement efficace permettant de prévenir ou traiter l'hypersensibilité à la douleur.

Par ailleurs, le document WO-A-02/22141 enseigne l'utilisation de xénon en tant que protecteur cardiovasculaire, sédatif, analgésique...

En outre, D. Ma et al, Xenon exerts age-independent antinociception in Fisher rats, mai 2004, Anesth., vol. 100, n°5, pages 1313-1318 montrent que l'action analgésique du xénon est indépendante de l'âge des patients, y compris chez le foetus ou le nouveau-né, alors que S. Pertersen-Felix et al, Comparison of the analgesic potency of xenon and nitrous oxide in humans evaluated by experimental pain NoV. 1998, Brit. J. of Anesth., vol. 81, n°5, p. 742-747, comparent les effets analgésiques du xénon à ceux du N₂O.

Un but de l'invention est d'améliorer l'efficacité de traitement ou de prévenir l'hypersensibilité à la douleur chez l'être humain, à savoir notamment l'homme, la femme, les enfants, les nouveaux-nés, et les personnes âgées, c'est-à-dire permettre d'éviter, de freiner ou diminuer l'apparition de ce phénomène néfaste d'hyperalgésie ou d'allodynie post-opératoire chez les patients.

Une solution selon l'invention est un mélange gazeux contenant de l'oxygène (O₂) et une proportion entre 20 et 70 % en volume de xénon (Xe) pour une utilisation en tant que médicament inhalé destiné à prévenir ou à traiter une hypersensibilité à la douleur post-opératoire ou post-traumatique chez l'humain ou l'animal, ladite hypersensibilité à la douleur se traduisant par l'apparition d'hyperalgésie ou d'allodynie.

Selon le cas, le mélange gazeux de l'invention peut comprendre l'une ou plusieurs des caractéristiques suivantes :
- la proportion de xénon est comprise entre 22 et 60 % en volume.
- la proportion de xénon est comprise entre 25 et 60 % en volume.
- il est constitué uniquement d'oxygène et de xénon ou d'air et de xénon.
- il contient, en outre, de l'azote, de l'hélium, du NO, du N₂O, du krypton, de l'argon ou du néon.
- il contient une proportion volumique d'oxygène comprise entre 15 et 25%.
- l'hypersensibilité à la douleur est de type post-opératoire ou post-traumatique et est induite par au moins un opioïde.
- il est conditionné à une pression supérieure à 2 bar au sein d'un récipient sous pression, notamment une bouteille de gaz.
- le mélange gazeux est conditionné sous une pression supérieure à 100 bar dans une bouteille de gaz. L'invention concerne aussi l'utilisation d'un mélange gazeux selon l'invention contenant de l'oxygène (O₂) et du xénon (Xe) en une proportion comprise entre 20 et 70 % en volume, pour fabriquer un médicament inhalable destiné à prévenir ou à diminuer une hyperalgésie chez l'humain ou l'animal.

L'invention porte par ailleurs aussi sur une méthode de traitement ou de prévention de l'hyperalgésie chez un patient comprenant l'étape d'administrer par inhalation audit patient, un mélange gazeux d'oxygène et de xénon selon l'invention.

Grâce au mélange gazeux de l'invention, on peut lutter efficacement contre le ou les effets néfastes d'une hyperalgésie post-opératoire ou post-traumatique.

L'invention va maintenant être mieux comprise grâce aux Exemples ci-dessous et à la description faite ci-après en relation avec les Figures annexées parmi lesquelles :
- la Figure 1A illustre (pour Xe 25%) le seuil de perception de la douleur par le rat quand une pression est opérée sur la patte enflammée, alors que la Figure 1B illustre (pour Xe 25%) ce seuil pour une patte non-enflammée.
- la Figure 2A illustre (pour Xe 50%) le seuil de perception de la douleur par le rat quand une pression est opérée sur la patte enflammée, alors que la Figure 2B illustre (pour Xe 50%) ce seuil pour une patte non-enflammée ;

### Exemples

Afin dé montrer l'efficacité du mélange gazeux selon l'invention dans la prévention des hyperalgésies post-traumatiques, un modèle de douleur inflammatoire a été utilisé chez le rat, à savoir des rats Sprague-Dawley mâles, dans le cadre d'études précliniques.

Un produit inflammatoire, le carragénine (0.2 ml de solution saline avec 1% de carragénine), a été injecté en sub-cutané dans la plante de la patte postérieure d'un rat.

Deux concentrations volumiques de xénon ont été testées, à savoir :
- un mélange gazeux ternaire formé de 25% Xe + 25% N₂ + 50% O₂ et
- un mélange gazeux binaire formé de 50% Xe + 50% O₂.

Le seuil nociceptif, i.e. le seuil de douleur, a été évalué en utilisant le test de vocalisation après pression de patte (Randall-Selitto), dans lequel des pressions croissantes (mesurées en grammes) sont appliquées à la patte arrière du rat jusqu'à ce que celui-ci se mette à crier.

Plus précisément, à Jour 0 (J0), le produit inflammatoire, i.e. le carragénine (Car), a été injecté dans une patte postérieure des rats.

L'exposition par inhalation des rats aux gaz débute 1h45 après l'injection de Car et dure 1h45. Le seuil nociceptive est évalué 1h30, 2h30, 3h00, 3h30 et 4h30 après l'injection de Car et puis une fois par jour jusqu'au retour au seuil nociceptif basal.

Les rats sont répartis en groupes comme suit:

| | Témoin | Invention |
|---|---|---|
| Essai 1 (Figure 1) | Carragénine / air | Carragénine / Xénon 25% |
| Essai 2 (Figure 2) | | Carragénine / Xénon 50% |

Après injection du produit inflammatoire (Car), les rats ont développé une hyperalgésie qui a duré 4 jours pour la patte enflammée et 2 jours pour la patte non enflammée. Cette hyperalgésie retardée résulte principalement d'un processus de sensibilisation à la douleur qui est NMDA-dépendant.

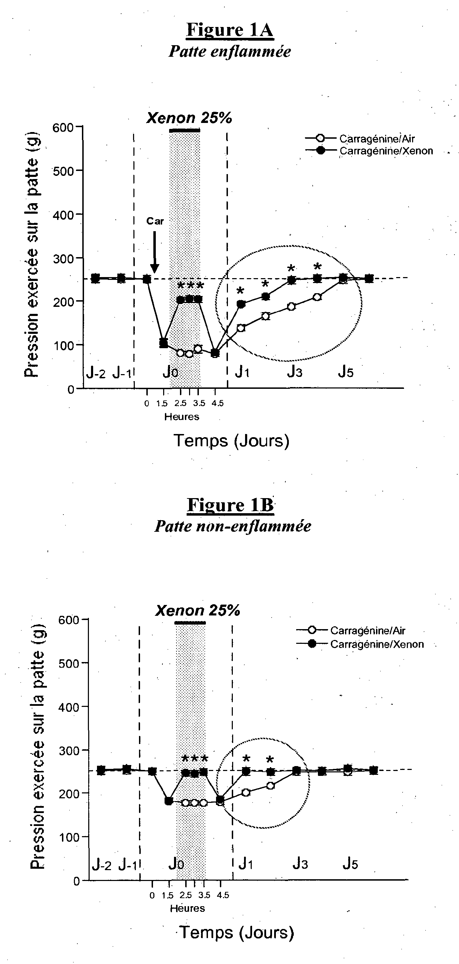

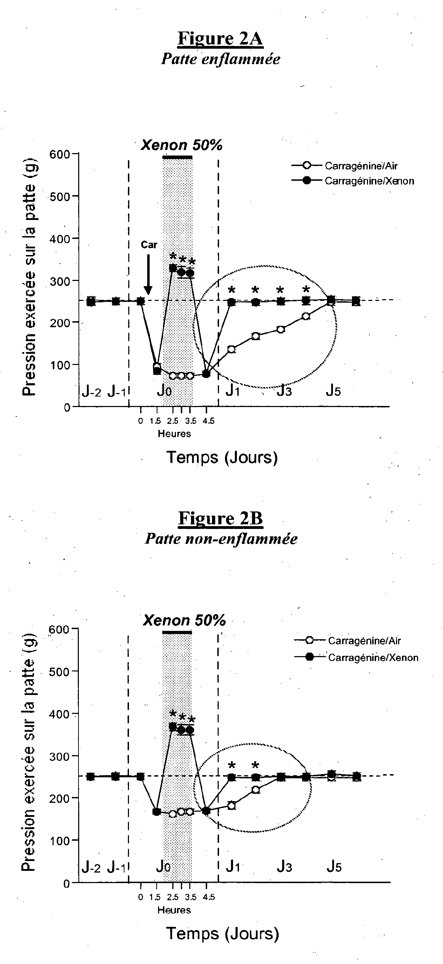

Les résultats obtenus (voir Figures) montrent que :
- le xénon a un effet analgésique pendant son administration et qu'une teneur de 50% (Fig. 2A) est plus efficace qu'une teneur de 25% (Fig. 1A).
- le xénon a un effet anti-hyperalgésique (cercle sur Figures) puisque :
   - le xénon à 25% réduit fortement l'hyperalgésie retardée sur la patte enflammée (Fig. 1A) : 2 jours contre 4 jours pour le témoin (air/Car).
   - le xénon à 50% prévient totalement l'hyperalgésie retardée sur les pattes enflammées et non-enflammées (Fig. 2A and 2B).

Ceci démontre que le xénon prévient le processus de sensibilisation à la douleur grâce à ses propriétés anti-hyperalgésiques vis-à-vis de l'hyperalgésie retardée au niveau de la patte enflammée.

De plus, le xénon prévient aussi complètement l'hyperalgésie retardée sur les pattes non enflammées, ce qui indique un effet central du xénon sur la sensibilisation à la douleur.

Le xénon est donc utilisable en tant que médicament préventif de l'hyperalgésie post-traumatique et de la douleur chronique.

Le mélange gazeux de l'invention contenant de l'oxygène O₂ et une proportion volumique de 20 à 70 % de xénon est donc utilisable dans le cadre d'une méthode de prévention ou de traitement thérapeutique, dans laquelle le mélange gazeux est administré par inhalation de manière à prévenir ou à traiter une hypersensibilité à la douleur chez l'humain ou l'animal, notamment chez l'homme, la femme ou l'enfant, en particulier d'une hyperalgésie post-opératoire ou post-traumatique.

## Revendications

1. Mélange gazeux contenant de l'oxygène (O₂) et une proportion volumique de 20 à 70 % de xénon (Xe) pour une utilisation en tant que médicament inhalable destiné à prévenir ou à traiter une hypersensibilité à la douleur post-opératoire ou post traumatique chez l'humain ou l'animal, ladite hypersensibilité à la douleur est du type s'exprimant par une hyperalgésie ou une allodynie.

2. Mélange gazeux pour une utilisation selon la revendication 1, **caractérisé en ce que** la proportion de xénon est comprise entre 22 et 60 % en volume.

3. Mélange gazeux pour une utilisation selon la revendication 2, **caractérisé en ce que** la proportion de xénon est comprise entre 25 et 60 % en volume.

4. Mélange gazeux pour une utilisation selon l'une des revendications 1 à 3, **caractérisé en ce qu'**il est constitué uniquement d'oxygène et de xénon ou d'air et de xénon.

5. Mélange gazeux pour une utilisation selon l'une des revendications 1 à 3, **caractérisé en ce qu'**il contient, en outre, de l'azote, de l'hélium, du NO, du krypton, de l'argon ou du néon.

6. Mélange gazeux pour une utilisation selon l'une des revendications 1 à 3 ou 5, **caractérisé en ce qu'**il contient une proportion volumique d'oxygène comprise entre 15 et 25%.

7. Mélange gazeux pour une utilisation selon l'une des revendications 1 à 6, **caractérisé en ce que** l'hypersensibilité à la douleur est induite par au moins un opioïde.

8. Mélange gazeux pour une utilisation selon l'une des revendications 1 à 7, **caractérisé en ce qu'**il est conditionné à une pression supérieure à 2 bar au sein d'un récipient sous pression, notamment une bouteille de gaz.

## Patentansprüche

1. Gasförmiges Gemisch, das Sauerstoff (O₂) und einen Volumenanteil an Xenon (Xe) von 20 bis 70 % enthält, für eine Verwendung als inhalierbares Arzneimittel zur Vorbeugung oder zur Behandlung einer postoperativen oder posttraumatischen Schmerzüberempfindlichkeit beim Menschen oder beim Tier, wobei die Schmerzüberempfindlichkeit des Typs ist, der sich in einer Hyperalgesie oder einer Allodynie ausdrückt.

2. Gasförmiges Gemisch für eine Verwendung nach Anspruch 1, **dadurch gekennzeichnet, dass** der Volumenanteil an Xenon zwischen 22 und 60 Volumen-% beträgt.

3. Gasförmiges Gemisch für eine Verwendung nach Anspruch 2, **dadurch gekennzeichnet, dass** der Volumenanteil an Xenon zwischen 25 und 60 Volumen-% beträgt.

4. Gasförmiges Gemisch für eine Verwendung nach einem der Ansprüche 1 bis 3, **dadurch gekennzeichnet, dass** es einzig aus Sauerstoff und aus Xenon oder aus Luft und aus Xenon besteht.

5. Gasförmiges Gemisch für eine Verwendung nach einem der Ansprüche 1 bis 3, **dadurch gekennzeichnet, dass** es ferner Stickstoff, Helium, NO, Krypton, Argon oder Neon enthält.

6. Gasförmiges Gemisch für eine Verwendung nach einem der Ansprüche 1 bis 3 oder 5, **dadurch gekennzeichnet, dass** es einen Volumenanteil an Sauerstoff zwischen 15 und 25 % enthält.

7. Gasförmiges Gemisch für eine Verwendung nach einem der Ansprüche 1 bis 6, **dadurch gekennzeichnet, dass** die Schmerzüberempfindlichkeit durch wenigstens ein Opioid bewirkt wird.

8. Gasförmiges Gemisch für eine Verwendung nach einem der Ansprüche 1 bis 7, **dadurch gekennzeichnet, dass** es bei einem Druck von über 2 bar in einem Druckbehälter, insbesondere einer Gasflasche, konditioniert wird.

## Claims

1. Gas mixture containing oxygen (O₂) and a proportion by volume of 20 to 70 % of xenon (Xe) for a use as an inhalable medicine which is intended to prevent or treat hypersensitivity to post-operative or post-traumatic pain in humans or animals, said hypersensitivity to pain being of the type expressed by hyperalgesia or allodynia.

2. Gas mixture for a use according to claim 1, **characterised in that** the proportion of xenon is between 22 and 60 % by volume.

3. Gas mixture for a use according to claim 2, **characterised in that** the proportion of xenon is between 25 and 60 % by volume.

4. Gas mixture for a use according to any of claims 1 to 3, **characterised in that** it consists only of oxygen and xenon or air and xenon.

5. Gas mixture for a use according to any of claims 1 to 3, **characterised in that** it also contains nitrogen, helium, NO, krypton, argon or neon.

6. Gas mixture for a use according to any of claims 1 to 3 or 5, **characterised in that** it contains a proportion by volume of oxygen of between 15 and 25 %.

7. Gas mixture for a use according to any of claims 1 to 6, **characterised in that** the hypersensitivity to pain is induced by at least one opioid.

8. Gas mixture for a use according to any of claims 1 to 7, **characterised in that** it is packaged at a pressure of greater than 2 bar inside a pressurised container, in particular a gas bottle.
